Europäisches Patentamt

European Patent Office

Office européen des brevets

(11) Veröffentlichungsnummer: **0 303 006 B1**

# (12) EUROPÄISCHE PATENTSCHRIFT

(45) Veröffentlichungstag der Patentschrift: **18.03.92**

(21) Anmeldenummer: **88107773.9**

(22) Anmeldetag: **14.05.88**

(51) Int. Cl.⁵: **A61F 2/34**

(54) Gelenkpfanne für eine Hüftgelenkendoprothese.

(30) Priorität: **13.08.87 DE 8711039 U**

(43) Veröffentlichungstag der Anmeldung:
**15.02.89 Patentblatt  89/07**

(45) Bekanntmachung des Hinweises auf die
Patenterteilung:
**18.03.92 Patentblatt  92/12**

(84) Benannte Vertragsstaaten:
**AT DE FR NL**

(56) Entgegenhaltungen:
**DE-A- 2 301 810      DE-A- 2 611 985
DE-U- 1 949 841      DE-U- 7 240 856
DE-U- 7 313 647      DE-U- 8 711 039
GB-A- 2 139 098**

(73) Patentinhaber: **Howmedica GmbH
Professor-Küntscher-Str. 1-5
W-2314 Schönkirchen ü. Kiel(DE)**

(72) Erfinder: **Seidel, Hartmut, Dr. med.
Reventlowstrasse 48
W-2000 Hamburg 52(DE)**
Erfinder: **Harder, Hans E.
Mecklenburger Strasse 37
W-2316 Probsteierhagen(DE)**

(74) Vertreter: **Dipl.-Ing. H. Hauck, Dipl.-Ing. E.
Graalfs, Dipl.-Ing. W. Wehnert, Dr.-Ing. W. Döring
Neuer Wall 41
W-2000 Hamburg 36(DE)**

Rank Xerox (UK) Business Services

**Beschreibung**

Die Erfindung bezieht sich auf eine Gelenkpfanne für eine Gelenkendoprothese nach dem Oberbegriff des Anspruchs 1.

Endoprothesen für den Ersatz des Hüftgelenks sind seit langer Zeit bekannt. Beispielhafte Ausführungen von Gelenkpfannen sind beispielsweise aus dem DE-GM 72 40 856 oder der DE-OS 26 11 985 bekanntgeworden. Die vorzugsweise aus Kunststoff geformten Pfannen werden mit Hilfe von Schrauben und von Knochenzement fixiert. Aus der DE-OS 23 01 810 ist auch bekanntgeworden, die Gelenkpfanne aus einer Außen- und einer Innenkappe herzustellen. Die Innenkappe ist lösbar in der Außenkappe untergebracht und nimmt ihrerseits die Gelenkkugel auf.

Nach längerer Implantationszeit besteht die Gefahr, daß der Knochenzement versprödet, zerbricht oder schrumpft. Aufgrund der ständigen einseitigen Belastung durch die Kugel kommt es zu einem Abarbeiten des Pfannendaches des natürlichen Acetabulums. Die Gelenkpfanne beginnt, sich zu lockern und muß entfernt werden.

Bei einer Reoperation kann eine übliche Gelenkpfanne wegen der zu geringen Außenabmessungen nicht mehr verwendet werden. Es ist daher auch bereits bekanntgeworden, verhältnismäßig große Ersatzpfannen vorzusehen mit einem Durchmesser von 60 bis 80 mm. Da derartige Pfannen ebenfalls mit Knochenzement fixiert werden, können nach einer gewissen Zeit erneut die gleichen Erscheinungen auftreten. Außerdem ist für den Einsatz einer derartig großen Gelenkpfanne notwendig, verhältnismäßig viel natürliches Knochenmaterial abzutragen. Es besteht daher die Gefahr, daß das Acetabulum nicht mehr die erforderliche Kraftaufnahme leistet und brechen kann. Ferner ist es problematisch, beim Verschrauben derartiger Revisionspfannen genügend Knochensubstanz zu finden für den wirksamen Eingriff des Schraubgewindes.

Aus der GB-A-2 139 098 ist eine Gelenkpfanne für eine Hüftgelenkendoprothese bekanntgeworden, die einen asymmetrischen Pfannenkörper aufweist. Der Pfannenkörper weist eine halbkugelige Höhlung auf, die im distalen Bereich abgeflacht ist. Die Außenseite des Pfannenkörpers ist konzentrisch zur halbkugeligen Höhlung geformt. Ein Einsatz aus Kunststoff für den Pfannenkörper weist ebenfalls eine halbkugelige Höhlung auf, die jedoch zur Halbkugeloberfläche der Außenseite exzentrisch liegt. Der Einsatz hat mithin eine unterschiedliche Wanddicke. Diese Ausbildung des Einsatzes dient zum leichteren Einsetzen in den Pfannenkörper. Der Pfannenkörper hat eine Reihe von Durchbrechungen für die Aufnahme einer Knochenschraube.

Der Erfindung liegt die Aufgabe zugrunde, eine Gelenkpfanne für eine Hüftgelenkendoprothese zu schaffen, die insbesondere als Ersatz für eine implantierte Gelenkpfanne bei ausgearbeitetem Pfannendach des Acetabulums geeignet ist.

Diese Aufgabe wird erfindungsgemäß gelöst durch die Merkmale des Kennzeichnungsteils des Anspruchs 1.

Die erfindungsgemäße Gelenkpfanne sieht einen länglich geformten Pfannenkörper vor, der senkrecht zur Achse der halbkugeligen Höhlung annähernd ein Oval bildet.Die halbkugelige Höhlung kann in dem Bereich liegen, in dem die anatomische Höhlung im Acetabulum gelegen hat.Der exzentrische mit einem größeren Durchmesser versehene Bereich erstreckt sich in den abgearbeiteten Bereich des Acetabulums in Richtung Pfannendach. Die erfindungsgemäße Gelenkpfanne nähert sich daher stark der Anatomie an, wie sie beim Abarbeiten des Pfannendachs durch eine prothetische Gelenpfanne entsteht. Es ist daher nur minimal Knochensubstanz abzuarbeiten, um die neuerungsgemäße Gelenkpfanne einzusetzen.

Da durch ihre Formgebung die Gelenkpfanne erhebliche Kräfte aufnehmen muß, besteht sie nach einer Ausgestaltung der Erfindung aus einem körperverträglichen Metall, beispielsweise rostfreiem Stahl, einer Kobaltchrommolybdänlegierung oder dergleichen.

Die erfindungsgemäße Gelenkpfanne wird ohne Knochenzement implantiert.Sie weist daher an der entsprechenden Oberfläche eine Struktur auf, die ein Verwachsen mit dem Knochenmaterial begünstigt. Eine Ausgestaltung der Erfindung sieht hierzu vor, daß die Außenfläche des Pfannenkörpers verkugelt ist. Die an die Außenfläche angeformten Kugeln haben zum Beispiel einen Durchmesser von 1,5 bis 2 mm, wobei die Kugeldichte etwa 11 bis 14 Stück pro cm$^2$ beträgt.

Insbesondere bei Herstellung des Pfannenkörpers aus Metall ist es vorteilhaft, wenn nach einer Ausgestaltung der Erfindung die halbkugelige Höhlung zur Aufnahme der Gelenkkugel von einem Kunststoffeinsatz gebildet ist, der klemmend in der angepaßten Höhlung des Pfannenkörpers festlegbar ist.

Die erfindungsgemäße Gelenkpfanne wird ebenfalls vorzugsweise mit Hilfe von Knochenschrauben im Acetabulum fixiert. Hierzu sieht eine Ausgestaltung der Erfindung vor, daß im Bereich einer durch die Achse der halbkugeligen Höhlung verlaufenden Längsebene jeweils mindestens eine Bohrung im Pfannenkörper vorgesehen ist zur Aufnahme einer Knochenschraube. Vorzugsweise sind zwei bis drei Bohrungen auf gegenüberliegenden Seiten geformt, die sich schräg nach oben und außen erstrecken. Die Bohrungen sind verhältnismäßig großzügig toleriert oder erstrecken sich konisch öffnend nach außen, so daß für die Kno-

2

chenschrauben ein Spielraum verbleibt, damit sie in eine günstige Partie des Acetabulums eingeschraubt werden können.

Bei entsprechender länglicher Ausdehnung des Pfannenkörpers ergibt sich eine relativ dicke Wand in dem zum Pfannendach zeigenden Bereich. Wenn dieser ausreichend dick ist, kann nach einer weiteren Ausgestaltung der Erfindung durch den dicken Wandbereich des Pfannenkörpers eine schräge Durchbohrung geformt sein, die an der Frontseite des Pfannenkörpers beginnt.

Damit eine sichere Auflage im Acetabulum erzielt wird, sieht eine weitere Ausgestaltung der Erfindung vor, daß der Pfannenkörper auf der der Frontseite gegenüberliegenden Seite eine Abflachung aufweist.

Die halbkugelige Höhlung im Pfannenkörper weist gegenüberliegend der Frontseite eine weitere Öffnung auf. Sie dient zur Kontrolle des paßgenauen Aufsitzes auf dem natürlichen Pfannengrund bzw. als Zugang zur Hinterfütterung mit Spongiosaspänen.

Die Erfindung wird nachfolgend anhand von Zeichnungen näher erläutert.

Fig. 1    zeigt eine Draufsicht auf eine Gelenkpfanne nach der Erfindung.

Fig. 2    zeigt einen Schnitt durch die Darstellung nach Fig. 1 entlang der Linie 2-2.

Fig. 3    zeigt einen Teil der Gelenkpfanne nach Fig. 2 in Richtung Pfeil 3.

Fig. 4    zeigt einen Schnitt durch die Darstellung nach Fig 1 entlang der Linie 4-4.

Fig. 5    zeigt einen Schnitt durch die Darstellung nach Fig. 1 entlang der Linie 5-5.

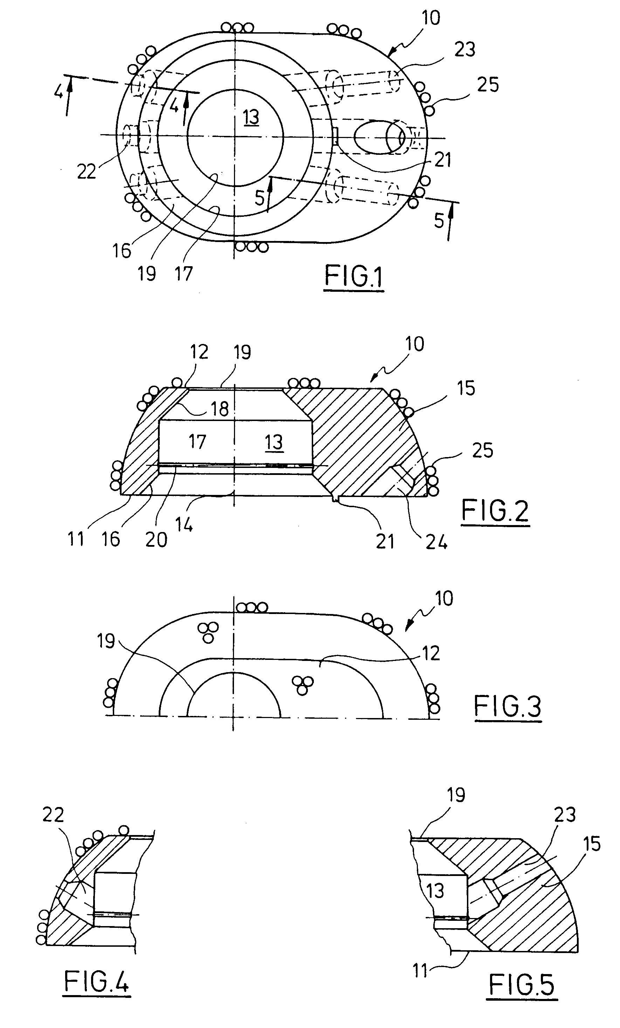

In den Figuren 1 bis 3 ist ein Pfannenkörper 10 zu erkennen, der einteilig aus Metall geformt ist, beispielsweise durch Gießformung. Als Werkstoff dient beispielsweise rostfreier Stahl, eine Kobaltchrommolybdänlegierung oder dergleichen.

Wie aus den Figuren 1 und 3 hervorgeht, ist der Pfannenkörper 10 im Umriß oval geformt. Seine Außenseiten sind kreisbogenförmig gekrümmt, wie aus den Figuren 2, 4 und 5 hervorgeht. Seine Frontseite 11 ist flach, ebenso seine Rückseite 12. Eine Höhlung von kreisförmiger Grundlinie weist eine Achse 14 auf, die, wie aus den Figuren 1 und 3 hervorgeht, außermittig liegt. Auf diese Weise ergibt sich ein relativ dicker Wandbereich 15, der auch als Ansatz eines im übrigen kreisförmigen Pfannenkörpers betrachtet werden kann. Die Höhlung 13 dient zur Aufnahme eines Pfanneneinsatzes aus geeignetem Kunststoffmaterial, dessen Außenkontur der Innenkontur der Höhlung 13 angepaßt ist. Die Höhlung 13 weist einen konischen Eingangsabschnitt 16, einen zylindrischen Mittelabschnitt 17 sowie einen sich konisch verjüngenden Abschnitt 18 auf. Der letztere endet in einer Öffnung 19. Im zylindrischen Abschnitt 17 ist eine Ringnut 20 geformt zur Aufnahme eines Sicherungsrings, der seinerseits in einer Ringnut an der Außenseite des nicht gezeigten Einsatzes sitzt, um den Einsatz in der Höhlung 13 zu fixieren. An der Frontfläche 11 befindet sich ein kleiner Vorsprung 21, der mit einer entsprechenden Ausnehmung des Einsatzes zusammenwirkt, um den Einsatz drehfest in der Höhlung 13 zu halten.

Wie aus den Figuren 1, 4 und 5 hervorgeht, sind sechs gestufte Bohrungen im Pfannenkörper 10 vorgesehen, wobei drei Bohrungen 22 sich durch den dem dickeren Abschnitt 15 gegenüberliegenden Wandbereich des Pfannenkörpers erstrecken und durch den dickeren Bereich die drei Bohrungen 23. Sie gehen vom zylindrischen Abschnitt 17 der Höhlung 13 aus und erstrecken sind schräg nach außen und hinten (Fig.4 und 5). Der innere Bereich weist einen größeren Durchmesser auf zur versenkten Aufnahme des Schraubenkopfes. Der andere Bereich der Bohrungen 22,23 ist so gewählt, daß die Schrauben mit Spiel nach außen öffnend geformt sind, um einen großen Bewegungsspielraum für die Knochenschrauben zu ermöglichen. Eine weitere Bohrung erstreckt sich von der Frontseite 11 im dickeren Bereich 14 schräg nach außen und innen. Sie ist mit 24 bezeichnet. Zu ihren Abmessungen gilt das gleiche wie zu den Bohrungen 22 und 23.

Die Außenfläche des Pfannenkörpers 10 ist verkugelt, wie bei 25 dargestellt. Die Kugeln können einen Durchmesser von 1,5 bis 2 mm aufweisen und in einer Dichte von 11 bis 14 Stück pro $cm^2$ angeordnet sein.

Der Pfannenkörper 10 wird im Acetabulum so eingesetzt, daß der dickere Bereich 15 in Richtung Pfannendach zeigt, mithin in dem abgearbeiteten Bereich des Acetabulums zu liegen kommt. Die Fixierung im Acetabulum erfolgt zementlos mit Hilfe der bereits beschriebenen Verschraubung und durch ein natürliches Einwachsen des Pfannenkörpers 15, das durch die Verkugelung 25 gefördert wird.

**Patentansprüche**

1.    Gelenkpfanne für eine Hüftgelenkendoprothese, mit einem Pfannenkörper (10), der auf der Frontseite eine halbkugelige Höhlung (13) zur Aufnahme einer Gelenkkugel aufweist, wobei die Achse der Höhlung (13) außermittig des Pfannenkörpers (10) liegt, dadurch gekennzeichnet, daß der Pfannenkörper (10) im Schnitt senkrecht zur außermittig liegenden Achse der Höhlung (13) länglich oval geformt ist.

2.    Gelenkpfanne nach Anspruch 1, dadurch gekennzeichnet, daß der Pfannenkörper (10) aus körperverträglichem Metall geformt ist.

3. Gelenkpfanne nach Anspruch 1 oder 2, dadurch gekennzeichnet, daß die halbkugelige Höhlung (13) von einem Kunststoffeinsatz gebildet ist, der klemmend in der angepaßten Höhlung (13) des Pfannenkörpers (10) festlegbar ist.

4. Gelenkpfanne nach Anspruch 3, dadurch gekennzeichnet, daß beidseitig einer durch die Achse (14) der halbkugeligen Höhlung (13) gehenden Längsebene jeweils mindestens eine Bohrung (22, 23) im Pfannenkörper (10) vorgesehen ist zur Aufnahme einer Knochenschraube.

5. Gelenkpfanne nach Anspruch 4, dadurch gekennzeichnet, daß die Bohrung (22, 23) im Durchmesser etwas größer ist als der Durchmesser der Schraube oder die Bohrung sich nach außen konisch erweitert.

6. Gelenkpfanne nach einem der Ansprüche 1 bis 5, dadurch gekennzeichnet, daß ein auf einer Seite der halbkugeligen Höhlung (13) liegender dickerer Wandbereich (15) eine schräge Durchbohrung (24) aufweist, die an der Frontseite (11) des Pfannenkörpers (10) beginnt.

7. Gelenkpfanne nach einem der Ansprüche 1 bis 6, dadurch gekennzeichnet, daß der Pfannenkörper (10) auf der der Frontseite (11) gegenüberliegenden Seite eine Abflachung (12) aufweist.

8. Gelenkpfanne nach einem der Ansprüche 3 bis 7, dadurch gekennzeichnet, daß die Höhlung (13) des Pfannenkörpers (10) auf der der Frontseite (11) gegenüberliegenden Seite eine Öffnung (19) aufweist.

9. Gelenkpfanne nach einem der Ansprüche 1 bis 8, dadurch gekennzeichnet, daß die Außenfläche des Pfannenkörpers (10) verkugelt (25) ist.

## Claims

1. A cup for a hip joint endoprosthesis comprising a cup member (10) having a hemispherical cavity (13) for the accommodation of a joint ball, with the axis of the cavity (13) eccentric with respect to the cup member (10), characterized in that the cross section of the cup member (10) perpendicular to the eccentric axis of the cavity (13) has an elongated oval shape.

2. The cup of claim 1, wherein the cup member (10) is made of a material compatible with the human body.

3. The cup of claim 1 or 2, wherein the hemispherical cavity (13) is provided by a plastic insert which can be fixedly clamped in the matched cavity of the cup member (10).

4. The cup of claim 3, wherein on both sides of a longitudinal plane extending through the axis (14) of the hemispherical cavity (13) at least one bore (22, 23) is provided in the cup member (10) for the accommodation of a bone screw.

5. The cup of claim 4, wherein the diameter of the bore (22, 23) is somewhat larger than the diameter of the screw or the bore enlarges conically outwardly, respectively.

6. The cup of one of the claims 1 to 5, wherein a thicker wall portion (15) on one side of the hemispherical cavity (13) includes an oblique throughbore (24) which starts at the front side (11) of the cup member (10).

7. The cup of one of the claims 1 to 6, wherein the cup member (10) has a flattening (12) on the side opposite to the front side (11).

8. The cup of one of the claims 3 to 7, wherein the cavity (13) of the cup member (10) has an opening (19) on the side opposite to the front side (11).

9. The cup of one of the claims 1 to 8, wherein the outer surface of the cup member (10) has a plurality of small balls.

## Revendications

1. Cupule acétabulaire pour une endoprothèse de la hanche, comportant un corps de cupule (10), qui présente, sur sa face frontale, un évidement (13) hémisphérique pour recevoir une tête d'articulation, l'axe de l'évidement (13) se trouvant excentré par rapport au corps de cupule (10), caractérisée en ce que le corps de cupule (10) a, en coupe perpendiculaire à l'axe en position excentrée de l'évidement (13), une forme ovale allongée.

2. Cupule acétabulaire suivant la revendication 1, caractérisée en ce que le corps de cupule (10) est moulé à partir de métal biologiquement compatible.

3. Cupule acétabulaire suivant la revendication 1 ou la revendication 2, caractérisée en ce que l'évidement (13) hémisphérique est pourvue d'une garniture insérée en matière plastique, qui peut être fixée et bloquée dans l'évidement (13), de forme ajustée du corps de cupule (10).

4. Cupule acétabulaire suivant la revendication 3, caractérisée en ce que, sur chacun des deux côtés d'un plan longitudinal passant par l'axe (14) de l'évidement hémisphérique (13), il est prévu au moins un alésage (22, 23) dans le corps de cupule (10) pour recevoir une vis de fixation dans l'os.

5. Cupule acétabulaire suivant la revendication 4, caractérisée en ce que l'alésage (22, 23) est d'un diamètre légèrement plus grand que le diamètre de la vis, ou bien que l'alésage s'élargit en formant un cône vers l'extérieur.

6. Cupule acétabulaire suivant l'une quelconque des revendications 1 à 5, caractérisée en ce que la zone de paroi plus épaisse (15), située sur un des côtés de l'évidement hémisphérique (13), présente un percement oblique (24), qui commence sur la face frontale (11) du corps de cupule (10).

7. Cupule acétabulaire suivant l'une quelconque des revendications 1 à 6, caractérisée en ce que le corps de cupule (10) présente un plat (12) sur sa face opposée à la face frontale.

8. Cupule acétabulaire suivant l'une quelconque des revendications 3 à 7, caractérisée en ce que l'évidement (13) du corps de cupule (10) présente une ouverture (19) sur sa face opposée à la face frontale.

9. Cupule acétabulaire suivant l'une quelconque des revendications 1 à 8, caractérisée en ce que la surface extérieure du corps de cupule (10) est munie de billes (25).

FIG.1

FIG.2

FIG.3

FIG.4

FIG.5